# EUROPÄISCHE PATENTSCHRIFT

(11) **EP 0 646 347 B1**
(45) Veröffentlichungstag und Bekanntmachung des Hinweises auf die Patenterteilung: **26.03.2003**
(21) Anmeldenummer: 94114385.1
(22) Anmeldetag: 13.09.1994
(51) Int. Cl.: A61B 8/00

(54) **Vorrichtung zur Aufnahme wenigstens einer sonographischen Sonde**
Device for reception of at least one sonography probe
Dispositif pour recevoir au moins une sonde de sonographie

(30) Priorität: 17.09.1993 DE 9314075 U
(43) Veröffentlichungstag der Anmeldung: 05.04.1995
(73) Patentinhaber: DWL Elektronische Systeme GmbH, D-78354 Sipplingen (DE)
(72) Erfinder: Lam, Arthur M., MD, Mercer Island, WA 98040 (US); Kempf, Friedemann, D-88709 Meersburg (DE)
(74) Vertreter: Hiebsch, Gerhard F., Dipl.-Ing.

(56) Entgegenhaltungen:
- DE-A- 4 131 430
- DE-C- 869 843
- DE-U- 8 908 141
- GB-A- 2 145 262

## Beschreibung

Die Erfindung betrifft eine Vorrichtung zur Aufnahme wenigstens einer sonographischen Sonde zu deren Einstellen und Festlegen am Schädel eines Patienten mit einem an diesem anbringbaren Gestell, an dem die Sonde in einem Sondenlager verstellbar ruht.

Eine derartige Vorrichtung ist der Schrift zum DE-GM 92 10 071 der Anmelderin zu entnehmen mit einer Halteplatte zur Fixierung am menschlichen Kopf, an der ein Sondenhalter verschiebbar und gegenüber der Halteplatte an beliebiger Stelle fixierbar gelagert ist. Dieser Sondenhalter umfaßt ein Haltestück für die Sonde, welches gegenüber der Halteplatte verschwenkbar im Sondenhalter lagert.

Aus der DE 41 31 430 A1 der Anmelderin ist ferner eine Vorrichtung nach dem Oberbegriff des Hauptanspruchs bekannt; eine derartige Vorrichtung löst das Problem einer individuellen Anpassung an den Schädel des Patienten durch die Verwendung von Bändern, die eine der Lagerung eines Sondenhalters dienende Halteplatte am menschlichen Kopf befestigen. Eine derartige Vorrichtung eignet sich zur exakten Justage der Sonde und ermöglicht es dem Patienten, die Sonde über einen längeren Zeitraum hinweg sicher zu tragen. Allerdings ist diese bekannte Vorrichtung im Hinblick auf das Anlegen bzw. Befestigen relativ aufwendig und mühsam.

In Kenntnis dieser Gegebenheiten hat sich der Erfinder das Ziel gesetzt, eine Halterung gattungsgemäßer Art weiterhin zu verbessern, vor allem im Hinblick auf die Anpassung an das Meßfenster des Schädels und der Einfachheit der Vorrichtung.

Zur Lösung dieser Aufgabe führt die Lehre des unabhängigen Anspruches; die Unteransprüche geben günstige Weiterbildungen an.

Erfindungsgemäß ist das Gestell brillenähnlich mit einem Nasenbügel und ein Paar an diesen beidseits anschließenden Haltebügeln ausgebildet, wobei der Abstand zwischen dem Nasenbügel und von den freien Enden des Gestells abragenden Ohrenbügeln oder entsprechenden Rastorganen einstellbar ist. An jedem Haltebügel lagern zwei streifenartige Tragzungen drehbar und längsverschieblich, welche einends miteinander durch ein an sie angelenktes Joch verbunden sind; dieses ist Teil des Sondenlagers.

Der Nasenbügel kann Teil des Gestelles oder in einer anderen Ausführung gegenüber dem Gestell -- zur Anpassung an die Schädelform des Benutzers -- abstandsveränderlich vorgesehen sein.

Die beiden Tragzungen verlaufen in Normallage etwa parallel zueinander und können beliebig in der Art der Seiten eines Parallelogrammes verschoben werden, an dessen einer Seite das Sondenlager bewegt wird. Ist das gewünschte Schädelfenster gefunden, genügt das Anziehen bereits einer Schraube, um das Sondenlager am Schädel festzulegen.

Nach einem weiteren Merkmal der Erfindung weist die Tragzunge einen Längsschlitz als Führung für einen Lagerstift des Seitenabschnittes auf, um den die Tragzunge drehbar und an dem sie zudem in ihrer gesamten Länge verschiebbar ist.

Damit das brillenähnliche Gestell am Schädel auf einfache Weise festgelegt zu werden vermag, ragen von den freien Enden der Seitenabschnitte jene Rastorgane als Ohrstöpsel ab, welche in den Gehörgang des Patienten eingeführt werden und zusammen mit dem Nasenbügel eine Dreipunktlagerung des Gestells am Schädel ermöglichen. Einer komplizierte Festlegung der Vorrichtung am Schädel bedarf es nicht mehr.

Ist der Nasenbügel Teil des Gestelles, sind dessen Enden beidseitig Teleskopstreifen zugeordnet, welche jene Ohrstöpsel enthalten; der Abstand von diesen zum Nasenbügel ist -- auch hier in Anpassung an die Schädelform des Benutzers -- dank der verschiebbaren Teleskopstreifen veränderbar.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
- Fig. 1, 7:: Frontansichten zweier Ausführungsformen einer erfindungsgemäßen brillenähnlichen Vorrichtung;
- Fig. 2, 8:: die Draufsicht auf Fig. 1 bzw. 7;
- Fig. 3, 9:: jeweils eine Seitenansicht zu Fig. 1, 2 und 7, 8;
- Fig. 4, 10:: jeweils die Innenansicht eines Teiles der Ausführung der Vorrichtung in einer Darstellung entsprechend Fig. 3 bzw. 9;
- Fig. 5:: ein Detail aus Fig. 1, 7 in vergrößerter und teilweise geschnittener Seitenansicht;
- Fig. 6:: die Frontansicht eines Teiles der Fig. 5;
- Fig. 11, 12:: Schrägsichten auf zwei unterschiedliche Ausführungen eines Abschnittes der Vorrichtung.

An einem brillenartigen Gestell 10 sind zwei zueinander gerichtete Sonden 12 für die medizinische Ultraschalldiagnostik des menschlichen Schädels in einem in der Zeichnung in etwa natürlicher Größe angedeuteten axialen Abstand a voneinander vorgesehen.

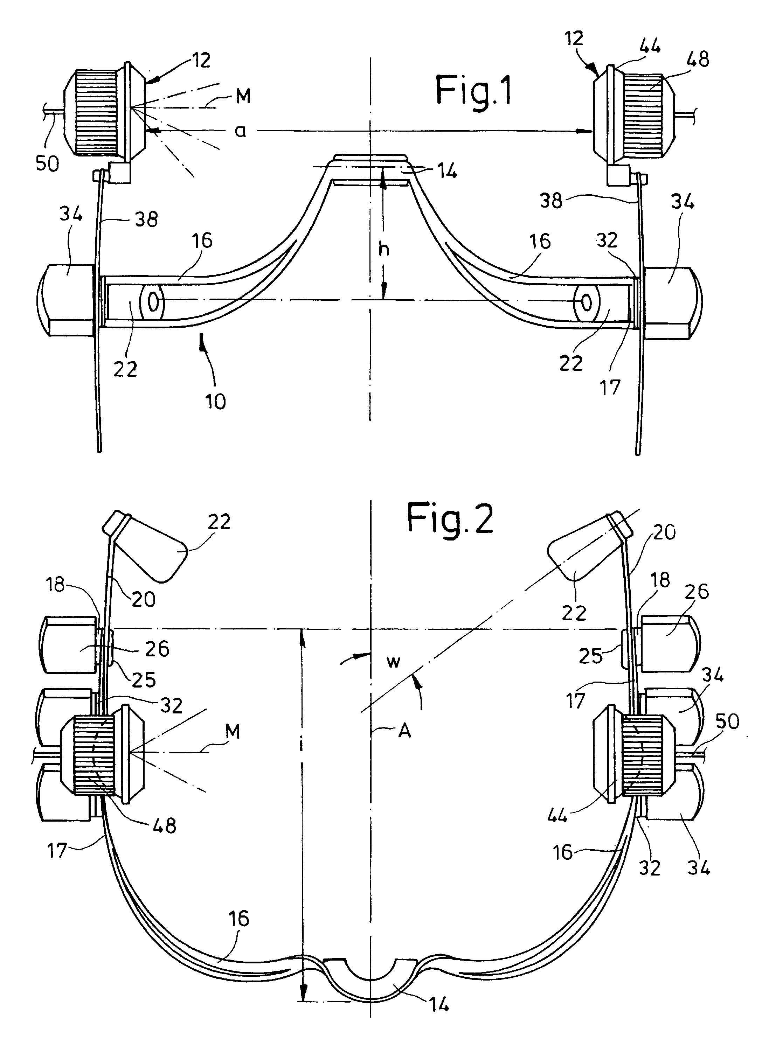

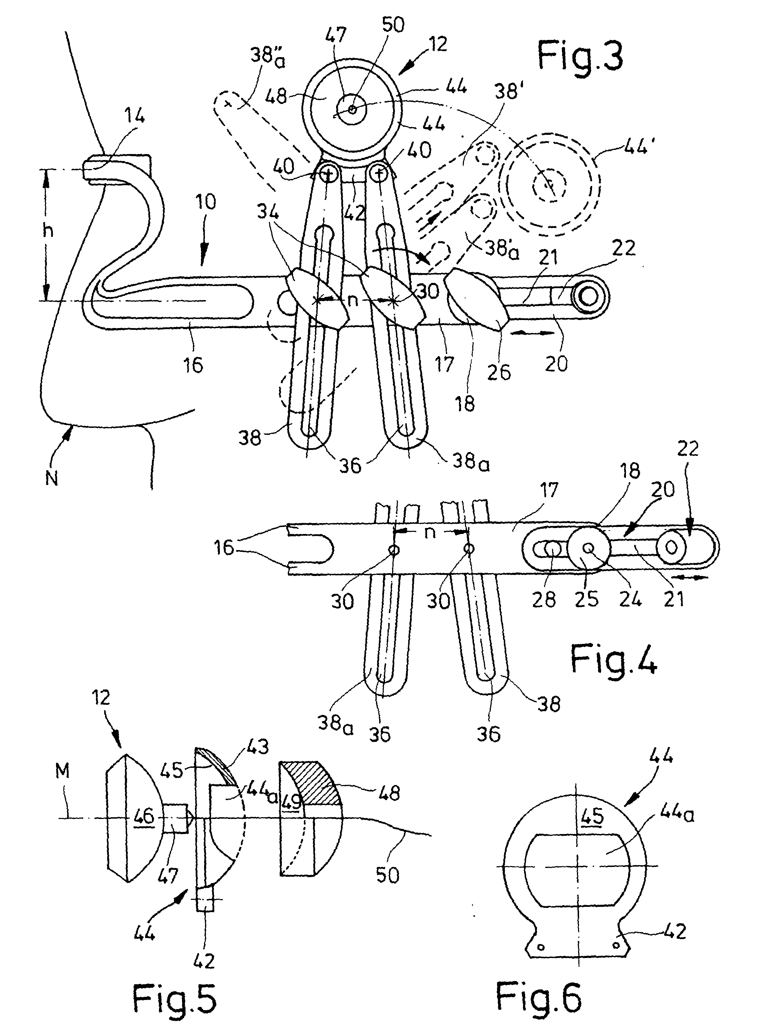

Das Gestell 10 weist einen von einer Symmetrieachse A gekreuzten Nasenbügel 14 für eine bei N skizzierten menschlichen Nase auf, an den beidseits jeweils ein Haltebügel 16 angeformt ist; dieser verläuft in Fig. 1 vom Nasenbügel 14 in einer Teilkreiskurve der Höhe h von beispielsweise 30 mm abwärts bis zu in Fig. 1, 3 horizontalen Bügelabschnitten der Haltebügel 16; diese enden als an die Schläfen des menschlichen Kopfes federnd anlegbare Seitenabschnitte 17. Diese gehen in Fig. 2 in einem horizontalen Abstand i von jenem Nasenbügel 14 in ein plattenartiges gerundetes Ende 18 über. An jedem dieser Seitenabschnitte 17 ist ein Teleskopstreifen 20 parallel verschiebbar angesetzt, der einen Ohrstöpsel 22 zur Befestigung des Gestells 10 am menschlichen Ohr dient. In der Befestigungslage des Gestells an drei Punkten -- an der menschlichen Nase N und den in der Zeichnung nicht dargestellten Ohren -- verlaufen die Seitenabschnitte 17 des Gestells 10 i.w. horizontal. Zur besseren Fixierung weist der Ohrstöpsel 22 in einem Winkel w mit der Symmetrieachse A zum Nasenbügel 14 hin (Fig. 2).

Einen Längsschlitz 21 des Teleskopstreifens 20 durchgreift ein Schraubbolzen 24, der sowohl in eine im Längsschlitz 21 verschiebbaren Gleitplatte 25 als auch -- an der äußeren Seite des Gestells 10 -- in einen Drehknopf 26 eingeschraubt ist und zum klemmenden Festlegen jenes Teleskopstreifens 20 am Seitenabschnitt 17 des Haltebügels 16 zwischen der Gleitplatte 25 und jener erwähnten plattenartigen Endausformung 18 dient. Außerdem gleitet im Längsschlitz 21 ein vom Haltebügel 16 abragender Führungsstift 28 zur parallelen Führung bei der Schubbewegung.

In jedem der Seitenabschnitte 17 sind in einem Abstand n voneinander zwei Lagerstifte 30 angebracht, welche jeweils eine plattenartige Anformung 32 der Haltebügel 16 durchsetzen und mit nach außen ragenden Schraubenden jeweils in einen Drehknopf 34 eingesetzt sind.

Jeder Lagerstift 30 durchsetzt den mittigen Längsschlitz 36 einer Tragzunge 38, 38a; beide sind um ihre Lagerstifte 30 drehbar und an ihnen längsverschieblich. Zudem sind sie an ihrem in der Zeichnung oberen Ende durch jeweils eine Schraube 40 an ein Joch 42 angelenkt.

Das Joch 42 ist Teil einer bevorzugt aus Kunststoff geformten Lagerschale 44 als Sondenlager - diese/s ähnelt einem Ring mit zentrischer Ausnehmung 44a und sphärischer Innenfläche 45, an die sich eine Teilkalottenfläche 46 der Sonde 12 anschmiegt und an der letztere somit verstellbar festliegt; mögliche Richtungen der Sondenmittelachse M sind in Fig. 1, 2 angedeutet. Die beiden Tragzungen 38, 38a bilden mit dem Joch 42 eine parallelogrammartige Verstelleinrichtung für das Sondenlager 44.

Ein in die Sondenmittelachse M fallender Schraubrohrstutzen 47 (Fig. 5) sitzt in einem Schraubring 48, der seinerseits mit einer sphärischen Innenfläche 49 der entsprechenden Außenfläche 43 der Lagerschale 44 und -- in deren zentrischer Ausnehmung 44a -- der Teilkalottenfläche 46 etwa anliegt. Durch jenen Schraubrohrstutzen 47 wird eine Sondenleitung 50 nach außen geführt, die in nicht dargestellter Weise an eine Auswerteinheit angeschlossen ist.

In Fig. 3 ist das Verschwenken der Verstelleinrichtung um den dort rechten Lagerstift 30 angedeutet: die Lagerzunge 38a wird gedreht und gelangt in die Stellung 38a', die andere Tragzunge 38 wird etwa parallel gedreht und gleichzeitig an ihrem Lagerstift 30 in Pfeilrichtung zu Position 38' verschoben, bis die Lagerschale 44 bei 44' steht.

Bei 38a' ist eine andere Stellung der Tragzunge 38a zu erkennen.

Dank der beschriebenen Lagerung der Sonde 12 kann diese am Kopf verstellt und auf die jeweiligen topographischen Verhältnisse des Patienten eingerichtet werden; für die Ultraschalldiagnostik im Inneren des Schädels muß an ihm die Stelle für die Sonde 12 gefunden werden, die eine transkraniale Untersuchung erlaubt.

Bei diesem Vorgang ist das eingeschaltete Auswert- oder Dopplergerät mit der Sonde 12 verbunden, das System für die Dopplersonographie also bereits in Funktion, so daß der Arzt an dem Dopplergerät genau verfolgen kann, an welcher für die beabsichtigte Ultraschalluntersuchung günstigen Stelle des Schädels sich die Sonde 12 gerade befindet; dort wird sie dann in beschriebener Weise durch Drehen an den Drehknöpfen 34 fixiert. Die Oberfläche der Sonde richtet sich dank der sphärischen Gleitflächen 45/46 und 43/49 selbst ein.

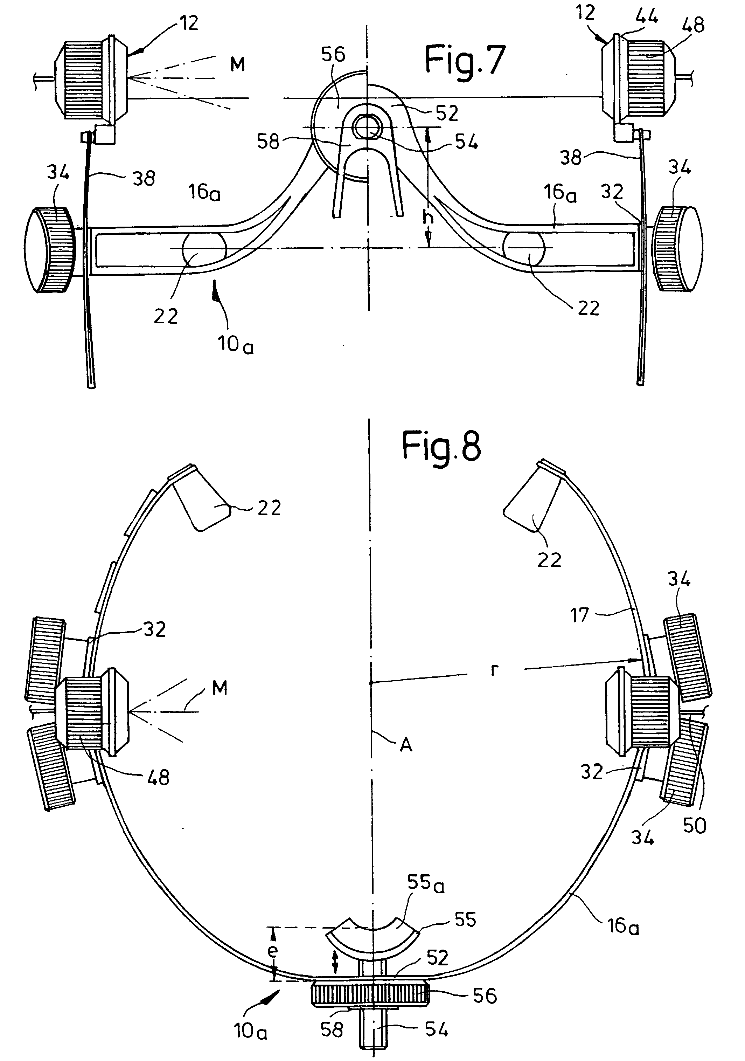

Bei der Ausführung nach den Fig. 7 bis 10 weist das Gestell 10a einen teilkreisförmigen Haltebügel 16a des Radius r auf. Dieser Haltebügel 16a ist einstückig, d.h. der Teleskopstreifen 20 der anderen Ausführung ist hier in den Haltebügel 16a fest integriert, die Ohrenstöpsel 22 sind fest mit dem Haltebügel 16a verbunden. Statt des Nasenbügels ist hier eine ebenfalls in den Haltebügel 16a integrierte Platte 52 (rechte Hälfte der Fig. 7) von einer undrehbar festliegenden Stellschraube 54 durchsetzt, die in einem Stellrad 56 verschoben werden kann. Letzteres ist zwischen jener Platte 52 und einem diese angeformten Haltesteg 58 vorgesehen. Die Stellschraube 54 endet an der Gestellinnenseite als Rinne 55 mit Schaumstoffeinlage 55a; diese wird auf die Nase N des Benutzers aufgelegt - das Gestell 10a ist dann dank der Stellschraube 54 auf einfache Weise zur Nase N abstandsveränderlich (Abstand e zwischen Stellrad 56 und Rinne 55).

Der Haltebügel 16a besteht aus zwei Randleisten 60, die einends zur Platte 52 sowie andernends zu den Ohrstöpseln 22 hin zusammenlaufen und zwischen sich einen Längsschlitz 62 bilden. Dieser ist in den Fig. 7 bis 11 durch eingeformte Ringe 64 unterbrochen, in welche Schraubbüchsen 66 von an den Bügelaußenseiten angeordneten Scheiben 69 eingesetzt sind. Deren jeweils mittiges Schraubloch 70 nimmt einen Lagerstift 30 auf.

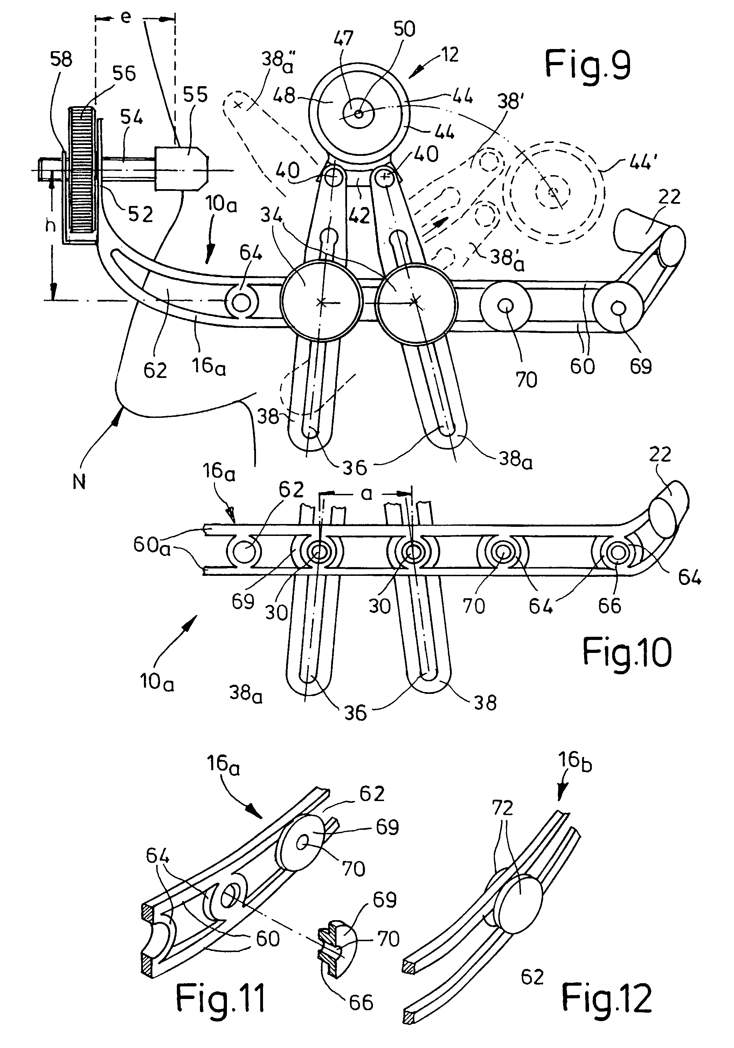

Bei der Ausführung nach Fig. 12 sind die Randleisten 60 nicht durch jene Ringe 64 verbunden; hier bildet der Längsschlitz 62 eine Führungsbahn für eine nicht dargestellte Stellschraube, die ein Paar von Klemmplatten 72 verbindet. Diese können mit anschließender Tragzunge 38, 38a stufenlos im Haltebügel 16b festgelegt werden.

## Patentansprüche

1. Vorrichtung zur Aufnahme wenigstens einer sonographischen Sonde (12) zu deren Einstellen und Festlegen am Schädel eines Patienten mit einem an diesem anbringbaren Gestell, an dem die Sonde (12) in einem Sondenlager (44) verstellbar ruht,
**dadurch gekennzeichnet,**
**daß** das Gestell (10, 10a) brillenähnlich einen Nasenbügel (14, 55) und an diesen beidseits anschließende Haltebügel (16, 16a) aufweist, wobei jeden Haltebügel zwei streifenartige Tragzungen (38, 38a) drehbar sowie längsverschieblich queren und diese Tragzungen einends miteinander durch ein an sie angelenktes Joch (42) verbunden sind, welches Teil des Sondenlagers (44) ist, wobei von den freien Enden des Gestells (10, 10a) jeweils ein stöpselartiger Querstift (22) abragt, der Abstand zwischen diesem und dem Nasenbügel (14, 15) veränderbar einzustellen ist und die stöpselartigen Querstifte (22) jeweils zum anderen Haltebügel (16, 16a) des Gestells (10, 10a) weisen.

2. Vorrichtung nach Anspruch 1, **dadurch gekennzeichnet, daß** die Tragzungen (38, 38a) jeweils einen Längsschlitz (36) als Führung für einen Lagerstift (30) des Haltebügels (16, 16a) aufweisen und an diesem klemmend festlegbar sind.

3. Vorrichtung nach Anspruch 2, **dadurch gekennzeichnet, daß** der Abstand (n) der Lagerstifte (30) voneinander etwa dem Abstand der Anlenkstellen (40) des Joches (42) entspricht oder die Tragzungen (38, 38a) mit dem Joch (42) am Haltebügel (16, 16a) des Gestells (10, 10a) parallelorgrammartig verstellbar sind.

4. Vorrichtung nach Anspruch 1, **dadurch gekennzeichnet, daß** der Nasenbügel (55) zum Gestell (10a) in seinem Abstand (e) veränderlich angeordnet ist, wobei gegebenenfalls der Nasenbügel (55) Teil eines Schraubelementes (54) ist, welches das Gestell (10a) durchsetzt und/oder am Gestell (10a) ein Schraubrad (56) drehbar lagert sowie von dem Schraubelement (54) durchsetzt ist.

5. Vorrichtung nach Anspruch 1, **dadurch gekennzeichnet, daß** der Haltebügel (16) einen Seitenabschnitt (17) aufweist sowie durch einen an diesem parallel verschieblich vorgesehenen Teleskopstreifen (20) verlängerbar ist, von dessen freien Ende der zum anderen Teleskopstreifen weisende stöpselartige Querstift (22) abragt.

6. Vorrichtung nach Anspruch 4 oder 5, **dadurch gekennzeichnet, daß** der stöpselartige Querstift(22) mit der Symmetrieachse (A) des Gestells (10) einen sich vom Nasenbügel (14) weg öffnenden Winkel (w) bestimmt.

7. Vorrichtung nach einem der Ansprüche 1 bis 6, **dadurch gekennzeichnet, daß** die Haltebügel (16, 16a) bzw. die Seitenabschnitte (17) des Gestells (10) zum Nasenbügel (14, 55) höhenversetzt (Höhe h) verlaufen.

8. Vorrichtung nach einem der Ansprüche 1 bis 7, **dadurch gekennzeichnet, daß** zwei Randleisten (60) der Haltebügel (16, 16a) einen Längsschlitz (62) begrenzen, wobei gegebenenfalls die Randleisten (60) durch in ihrer Ebene liegende Ringe (64) verbunden sind oder eine Führungsbahn für Klemmeinrichtungen (30, 72) bilden.

9. Vorrichtung nach wenigstens einem der Ansprüche 1 bis 8 mit einem wenigstens eine teilsphärische Fläche aufweisenden Sondenlager (44) zur Anlage an einer entsprechend geformten Gleitfläche der daran bewegbaren Sonde, wobei das Joch (42) Teil des Sondenlagers (44) und dieses mit einer zentrischen Ausnehmung (44a) versehen ist.

10. Vorrichtung nach wenigstens einem der Ansprüche 1 bis 9, **dadurch gekennzeichnet, daß** das Sondenlager (44) in der Art eines Ringes mit sphärischen Seitenflächen (43, 45) ausgebildet ist, wobei einer dieser Seitenflächen die Sonde (12) und der anderen ein mit der Sonde verbindbarer Schraubring (48) benachbart ist.

11. Vorrichtung nach Anspruch 10, **dadurch gekennzeichnet, daß** Sonde (12) und Schraubring (48) durch ein rohrartiges Element (47) verbunden und dieses in der zentrischen Ausnehmung (44a) verschiebbar ist.

## Claims

1. Device for receiving at least one sonography probe (12) for the adjustment and fixing thereof on the skull of a patient, comprising a frame which can be arranged thereon and on which the probe (12) adjustably rests in a probe bearing (44), **characterised in that** the frame (10, 10a), similar to a pair of spectacles, comprises a nose piece (14, 55) and holding pieces (16, 16a) adjoining it on either side, two strip-like carrying tongues (38, 38a) rotatably and longitudinally displaceably crossing each holding piece and these carrying tongues being connected to one another at one end by a yoke (42) articulated thereto and which is part of the probe bearing (44), a respective stopper-like transverse pin (22) projecting from the free ends of the frame (10, 10a), the spacing between this and the nose piece (14, 15) being variably adjustable and the stopper-like transverse pins (22) each pointing to the other holding piece (16, 16a) of the frame (10, 10a).

2. Device according to claim 1, **characterised in that** the carrying tongues (38, 38a) each comprise a slot (36) as a guide for a bearing pin (30) of the holding piece (16, 16a) and can be fixed thereto in a clamping manner.

3. Device according to claim 2, **characterised in that** the spacing (n) of the bearing pins (30) from one another approximately corresponds to the spacing of the articulation points (40) of the yoke (42), or the bearing tongues (38, 38a) are adjustable in a parallelogram-like manner on the holding piece (16, 16a) of the frame (10, 10a) with the yoke (42).

4. Device according to claim 1, **characterised in that** the nose piece (55) is arranged at an adjustable spacing (e) from the frame (10a), the nose piece (55) optionally being part of a screw element (54) penetrating the frame (10a) and/or a spiral-toothed gear wheel (56) being rotatably mounted on the frame (10a) and being penetrated by the screw element (54).

5. Device according to claim 1, **characterised in that** the holding piece (16) comprises a side portion (17) and is extendable owing to a telescopic strip (20) which is displaceably provided in parallel thereon and from the free end of which the stopper-like transverse pin (22) pointing coward the other telescopic strip projects.

6. Device according to claim 4 or 5, **characterised in that** the stopper-like transverse pin (22) determines, with the axis of symmetry (A) of the frame (10), an angle (w) opening away from the nose piece (14).

7. Device according to any of claims 1 to 6, **characterised in that** the holding pieces (16, 16a) and the side portions (17) of the frame (10) extend offset in height (height h) from the nose piece (14, 55).

8. Device according to any of claims 1 to 7, **characterised in that** two edge strips (60) of the holding pieces (16, 16a) delimit a slot (62), the edge strips (60) optionally being connected via rings (64) located in their plane or forming a guide path for fixing devices (30, 72).

9. Device according to at least any one of claims 1 to 8, comprising a probe bearing (44) with an at least partially spherical face for abutment on a correspondingly formed sliding face of the probe which can be moved thereon, the yoke (42) being part of the probe bearing (44) and the latter being provided with a central recess (44a).

10. Device according to at least any of claims 1 to 9, **characterised in that** the probe bearing (44) is constructed in the manner of a ring with spherical side faces (43, 45), one of these side faces being adjacent to the probe (12) and the other being adjacent to the threaded ring (48) connectable to the probe.

11. Device according to claim 10, **characterised in that** probe (12) and threaded ring (48) are connected by a tubular element (47) and the latter can be displaced in the central recess (44a).

## Revendications

1. Dispositif pour recevoir au moins une sonde sonographique (12), destiné au réglage et à la fixation de cette dernière sur le crâne d'un patient, comprenant une monture pouvant être mise en place sur celui-ci, sur laquelle repose la sonde (12) de façon réglable dans un logement de sonde (44), **caractérisé en ce que** la monture (10, 10a) présente, à l'image des lunettes, un étrier de nez (14, 55) et des étriers de maintien (16, 16a) prolongeant ce dernier aux deux côtés, chaque étrier de maintien étant traversé de façon pivotante et mobile dans le sens longitudinal par deux languettes de support (38, 38a) en forme de bande, et ces languettes de support étant reliées à une extrémité l'une à l'autre par une travée (42) articulée sur celles-ci et qui est solidaire du logement de sonde (44), des extrémités libres de la monture (10, 10a) une goupille transversale (22) en forme de bouchon saillant respectivement, la distance entre celle-ci et l'étrier de nez (14, 15) étant réglable, et les goupilles transversales (22) en forme de bouchon étant respectivement orientées vers l'autre étrier de maintien (16, 16a) de la monture (10, 10a).

2. Dispositif selon la revendication 1, **caractérisé en ce que** les languettes de support (38, 38a) présentent respectivement une fente oblongue (36) servant à guider une goupille de logement (30) de l'étrier de maintien (16, 16a) et **en ce qu'**elles peuvent être fixées sur celui-ci par serrage.

3. Dispositif selon la revendication 2, **caractérisé en ce que** la distance (n) des goupilles de logement (30) l'une par rapport à l'autre correspond sensiblement à la distance des points d'articulation (40) de la travée (42), ou **en ce que** les languettes de support (38, 38a) avec la travée (42) sont réglables sur l'étrier de maintien (16, 16a) de 1a monture (10, 10a) à la manière d'un parallélogramme.

4. Dispositif selon la revendication 1, **caractérisé en ce que** l'étrier de nez (55) est disposé réglable au niveau de sa distance (e) par rapport à la monture (10a), l'étrier de nez (55) faisant le cas échéant partie d'un élément fileté (54) qui traverse la monture (10a), et/ou une roue dentée (56) étant logée de manière pivotante sur la monture (10a) et étant traversée par l'élément fileté (54).

5. Dispositif selon la revendication 1, **caractérisé en ce que** l'étrier de maintien (16) présente un tronçon latéral (17) et peut être prolongé à l'aide d'une bande télescopique (20) prévue sur celui-ci à déplacement parallèle, de l'extrémité libre duquel saille la goupille transversale (22) orientée vers l'autre bande télescopique.

6. Dispositif selon la revendication 4 ou 5, **caractérisé en ce que** la goupille transversale (22) en forme de bouchon définit avec l'axe symétrique (A) de la monture (10) un angle (w) ouvert dans le sens opposé à l'étrier de nez (14).

7. Dispositif selon l'une des revendications 1 à 6, **caractérisé en ce que** les étriers de maintien (16, 16a) ou les tronçons latéraux (17) de la monture (10) s'étendent décalés en hauteur (hauteur h) par rapport à l'étrier de nez (14, 55).

8. Dispositif selon l'une des revendications 1 à 7, **caractérisé en ce que** deux lisières (60) des étriers de maintien (16, 16a) délimitent une fente oblongue (62), les lisières (60) étant le cas échéant reliées à l'aide d'anneaux (64) situés à leur niveau, ou constituant un chemin de guidage pour des dispositifs de serrage (30, 72).

9. Dispositif selon au moins l'une des revendications 1 à 8, comprenant un logement de sonde (44) présentant au moins une surface partiellement sphérique pour l'application sur une surface de glissement, formée en conséquence, de la sonde pouvant être déplacée sur celle-ci, la travée (42) faisant partie du logement de sonde (44) qui présente un creux central (44a).

10. Dispositif selon au moins l'une des revendications 1 à 9, **caractérisé en ce que** le logement de sonde (44) est se présente sous la forme d'un anneau comportant des faces latérales sphériques (43, 45), dans lequel la sonde (12) est voisine de l'une des faces latérales et un anneau fileté (48) pouvant être relié à la sonde est voisin de l'autre face latérale.

11. Dispositif selon la revendication 10, **caractérisé en ce que** la sonde (12) et l'anneau fileté (48) sont reliés par un élément tubulaire (47) qui peut être déplacé à l'intérieur du creux central.
